# EUROPÄISCHE PATENTSCHRIFT

(11) **EP 2 794 552 B1**
(45) Veröffentlichungstag und Bekanntmachung des Hinweises auf die Patenterteilung: **11.04.2018**
(21) Anmeldenummer: 12809165.9
(22) Anmeldetag: 12.12.2012
(51) Int. Cl.: C07C 227/08

(54) **VERFAHREN ZUR HERSTELLUNG VON RACEMISCHEN A-AMINOSÄUREN**
METHOD FOR MANUFACTURING RACEMIC ALPHA AMINO ACIDS
PROCÉDÉ DE FABRICATION D'ACIDES ALPHA-AMINÉS RACÉMIQUES

(30) Priorität: 19.12.2011 EP 11194363; 09.07.2012 EP 12175552
(43) Veröffentlichungstag der Anmeldung: 29.10.2014
(73) Patentinhaber: BASF SE, 67056 Ludwigshafen am Rhein (DE)
(72) Erfinder: BOU CHEDID, Roland, 68159 Mannheim (DE); OFTRING, Alfred, 67098 Bad Dürkheim (DE); STAFFEL, Wolfgang, 67166 Otterstadt (DE); BIEL, Markus Christian, 68159 Mannheim (DE); MELDER, Johann-Peter, 67459 Böhl-Iggelheim (DE); GRÜNANGER, Christian, 68163 Mannheim (DE)
(74) Vertreter: BASF IP Association
(86) Internationale Anmeldenummer: PCT/EP2012/075180
(87) Internationale Veröffentlichungsnummer: WO 2013/092329

(56) Entgegenhaltungen:
- EP-A1- 0 295 550
- US-A- 4 350 826
- US-A1- 2004 092 725
- US-A1- 2004 162 434

## Beschreibung

Die vorliegende Erfindung betrifft ein Verfahren zur Herstellung von racemischen α-Aminosäuren oder von Glycin, das dadurch gekennzeichnet ist, dass man die korrespondierende α-Hydroxycarbonsäure, gewählt aus Hydroxyessigsäure, Milchsäure, Äpfelsäure, α-Hydroxyglutarsäure, Isocitronensäure, Tartronsäure und Weinsäure, oder mindestens ein Salz der korrespondierenden α-Hydroxycarbonsäure in Gegenwart von mindestens einem heterogenen Katalysator, der mindestens ein Übergangsmetall enthält, in Gegenwart von Wasserstoff mit mindestens einer Stickstoffverbindung (c) umsetzt, wobei man Stickstoffverbindung (c) wählt aus primären und sekundären Aminen und Ammoniak.

Weiterhin betrifft die vorliegende Erfindung Gemische von bestimmten racemischen α-Aminosäuren und den korrespondierenden α-Hydroxycarbonsäuren.

Weiterhin betrifft die vorliegende Erfindung die Verwendung von erfindungsgemäßen Gemischen.

Aminosäuren haben zahlreiche Anwendungsgebiete. So werden L-Aminosäuren zur Peptid- und zur Proteinsynthese eingesetzt. Doch auch racemische Aminsäuren sind begehrte Zwischenprodukte.

Die Herstellung von racemischen Aminosäuren durch die Streckersynthese ist an sich bekannt. Nachteilig ist, dass man mit Blausäure bzw. den entsprechenden Cyaniden sehr toxische Substanzen benötigt, die besondere Sicherheitsvorkehrungen notwendig machen.

Aus US 2004/092725 ist ein Verfahren bekannt, α-Aminosäuren aus korrespondierenden α-Hydroxycarbonsäuren zu synthetisieren, indem man mit Ammoniak bei hohem Druck und vorzugsweise mindestens 300°C umsetzt. Die Ausbeuten sind allerdings gering. So wird die Synthese von Glycin bei 374°C mit einer Ausbeute von 4,3% und die Synthese von α-Alanin bei 374°C mit einer Ausbeute von 2,8% offenbart. Derartige Ausbeuten sind in großtechnischen Verfahren allerdings unbefriedigend. Außerdem sind die erhaltenen Produkte meist dunkel verfärbt und erfordern dann ein aufwändiges Reinigungsverfahren.

Es bestand also die Aufgabe, ein Verfahren bereit zu stellen, durch das man α-Aminosäuren in guten Ausbeuten gewinnen kann. Es bestand weiterhin die Aufgabe, Vorstufen für die Herstellung von Mischungen von Wasch- und Reinigungsmitteln einschließlich Mitteln für die maschinelle Geschirrreinigung bereit zu stellen, die eine geringe oder gar nicht ausgebildete Tendenz zum Verklumpen aufweisen.

Dementsprechend wurde das eingangs definierte Verfahren gefunden, im Rahmen der vorliegenden Erfindung auch erfindungsgemäßes Verfahren genannt.

Zur Durchführung des erfindungsgemäßen Verfahrens geht man aus von mindestens einer α-Hydroxycarbonsäure, beispielsweise von einem Gemisch von zwei oder drei α-Hydroxycarbonsäuren oder von einer α-Hydroxycarbonsäure. α-Hydroxycarbonsäure kann man in enantiomerenreiner Form einsetzen oder als Mischung von Enantiomeren, beispielsweise als Racemat, so verschiedene Enantiomeren von der betreffenden α-Hydroxycarbonsäure existieren.

α-Hydroxycarbonsäure kann man als freie Säure einsetzen oder in partiell oder vollständig neutralisierter Form. Wenn man α-Hydroxycarbonsäure in vollständig oder partiell neutralisierter Form einsetzen möchte, so sind Ammoniumsalze und Alkalimetallsalze wie beispielsweise Kaliumsalze und insbesondere Natriumsalze bevorzugt.

Beispiele für α-Hydroxycarbonsäuren sind Hydroxyessigsäure, Milchsäure, insbesondere L-Milchsäure, Äpfelsäure, insbesondere L-Äpfelsäure, und α-Hydroxyglutarsäure, weiterhin Isocitronensäure, Tartronsäure und Weinsäure. Besonders bevorzugte Beispiele sind racemische Milchsäure, (-)-Milchsäure und (+)-Milchsäure.

Im Falle der Herstellung von Glycin, welches nicht chiral ist, erhält man kein Racemat, sondern Glycin.

In einer Ausführungsform der vorliegenden Erfindung führt man das erfindungsgemäße Verfahren in wässrigem Milieu durch. Darunter ist zu verstehen, dass man α-Hydroxycarbonsäure oder ihr Salz in Wasser suspendiert oder löst oder in einem Gemisch, das zu mindestens 75 Vol.-% Wasser enthält und insgesamt bis zu 25 Vol.-% organisches Lösungsmittel enthalten kann, beispielsweise Tetrahydrofuran oder N,N-Dimethylformamid, wobei Vol.-% auf gesamte kontinuierliche Phase bezogen ist. Alkohole sind als organische Lösungsmittel nicht geeignet. Vorzugsweise setzt man kein organisches Lösungsmittel oder nur 0,1 bis 5 Vol.-% organisches Lösungsmittel ein, bezogen auf kontinuierliche Phase.

In einer Ausführungsform der vorliegenden Erfindung führt man das erfindungsgemäße Verfahren bei einem pH-Wert im Bereich von 4 bis 14 durch, bevorzugt 6 bis 14 und besonders bevorzugt 8 bis 13,5.

Erfindungsgemäß setzt man mit mindestens einer Stickstoffverbindung (c) um, wobei man Stickstoffverbindung (c) wählt aus primären Aminen, bevorzugt sekundären Aminen und noch mehr bevorzugt Ammoniak. Beispiele für primäre Amine sind insbesondere C₁-C₁₀-Alkylamine, beispielsweise Methylamin, Ethylamin, Isopropylamin, tert.-Butylamin, n-Decylamin, weiterhin aromatische Amine wie Anilin, C₃-C₇-Cycloalkylamine wie beispielsweise Cyclohexylamin und Monoethanolamin. Beispiele für sekundäre Amine sind Di-C₁-C₁₀-Alkylamine, insbesondere Dimethylamin, Diethylamin und Diisopropylamin, weiterhin Di-C₂-C₄-hydroxyalkylenamine, insbesondere Diethanolamin, weiterhin Mono-C₁-C₁₀-alkylmono-C₂-C₄-hydroxyalkylenamine, beispielsweise N-Methyl-N-ethanolamin, weiterhin cyclische sekundäre Amine wie Piperidin und Morpholin. Weitere geeignete Stickstoffverbindungen (c) sind Iminodicarbonsäuren, insbesondere Iminodiessigsäure.

In einer bevorzugten Ausführungsform der vorliegenden Erfindung wählt man als Stickstoffverbindung (c) Ammoniak, das heißt man setzt mit Ammoniak um. Ammoniak kann man der Reaktionsmischung in Form von flüssigem Ammoniak, gasförmigem Ammoniak oder Ammoniakwasser ("NH₄OH") zusetzen. Wünscht man Ammoniak in Form eines Ammoniumsalzes zuzusetzen, so ist es bevorzugt, Ammoniak in Kombination mit mindestens einer starken Base zuzusetzen, beispielsweise in Kombination mit NaOH oder KOH. Bevorzugt ist es, Ammoniak in Form von flüssigem Ammoniak oder Ammoniakwasser zuzusetzen.

In einer Ausführungsform setzt man α-Hydroxycarbonsäure und Stickstoffverbindung (c) in einem Molverhältnis im Bereich von 1 : 1 bis 1 : 100 ein, bevorzugt im Bereich von 1 : 2 bis 1 : 50, besonders bevorzugt im Bereich von 1 : 3 bis 1 : 30. Dabei bezieht sich der Anteil an Stickstoffverbindung (c) auf die Summe aller Stickstoffverbindungen (c).

In einer Ausführungsform setzt man α-Hydroxycarbonsäure und Ammoniak in einem Molverhältnis im Bereich von 1 : 1 bis 1 : 100 ein, bevorzugt im Bereich von 1 : 2 bis 1 : 50, besonders bevorzugt im Bereich von 1 : 3 bis 1 : 30.

Das erfindungsgemäße Verfahren führt man in Gegenwart von Wasserstoff durch, also von H₂. In einer Ausführungsform der vorliegenden Erfindung führt man das erfindungsgemäße Verfahren so durch, dass man insgesamt ein Molverhältnis α-Hydroxycarbonsäure zu Wasserstoff im Bereich von 1:1 bis 1:90, bevorzugt 1:2 bis 1:30 einstellt. In einer anderen Ausführungsform stellt man ein Molverhältnis von α-Hydroxycarbonsäure zu Wasserstoff im Bereich von 2:1 bis 1,01:1 ein.

In einer Ausführungsform der vorliegenden Erfindung kann man Wasserstoff durch ein unter den Reaktionsbedingungen des erfindungsgemäßen Verfahrens inertes Gas verdünnen, beispielsweise mit Stickstoff oder mit mindestens einem Edelgas, beispielsweise mit Argon.

Das erfindungsgemäße Verfahren führt man in Gegenwart von mindestens einem heterogenen Katalysator durch, der mindestens ein Übergangsmetall enthält. Als heterogene Katalysatoren können dabei dienen:
(i) auf einem festen Träger, der in partikulärer Form vorliegt, geträgerte Metall-haltige Katalysatoren,
(ii) auf einem festem Träger, der in nicht-partikulärer Form vorliegt, geträgerte Metall-haltige Katalysatoren,
(iii) Träger-freie katalytisch aktive Partikel.

Im Rahmen der vorliegenden Erfindung umfasst dabei der Begriff Katalysator dabei Übergangsmetall, das als katalytisch aktive Spezies ("Haupt-Metall") dient, oder gegebenenfalls eine Vorstufe davon, weiterhin gegebenenfalls vorhandener Träger und gegebenenfalls vorhandene Dotierung.

Unter "festem Träger" sollen dabei solche Materialien verstanden werden, die unter den Reaktionsbedingungen des erfindungsgemäßen Verfahrens fest sind und die für die Formgebung des heterogenen Katalysators maßgeblich sind.

Unter "in partikulärer Form vorliegend" soll verstanden werden, dass der betreffende Träger in Form von Partikeln vorliegt, deren durchschnittlicher Durchmesser im Bereich von 0,1 µm bis 2 mm liegt, bevorzugt 0,001 bis 1 mm, bevorzugt im Bereich von 0,005 bis 0,5 mm, insbesondere 0,01 bis 0,25 mm.

Unter "in nicht-partikulärer Form vorliegende" soll verstanden werden, dass der Träger in mindestens einer Dimension (Breite, Höhe, Tiefe) mehr als 2 mm, bevorzugt mindestens 5 mm aufweist, wobei mindestens eine weitere Dimension, beispielsweise ein oder beide weiteren Dimensionen, kleiner als 2 mm groß sein kann, beispielsweise im Bereich von 0,1 µm bis 2 mm. In einer anderen Variante weist in nicht-partikulärer Form vorliegender Träger drei Dimensionen auf, die eine Abmessung von mehr als 2 mm, bevorzugt mindestens 5 mm, aufweisen. Als Obergrenze sind beispielsweise 10 m, bevorzugt 10 cm geeignet.

Beispiele für Träger, die in nicht-partikulärer Form vorliegen, sind Metallnetze, beispielsweise Stahlnetze oder Nickelnetze, weiterhin Drähte wie Stahldrähte oder Nickeldrähte, weiterhin Formkörper, beispielsweise Kugeln, Raschig-Ringe, Stränge und Tabletten.

In einer Ausführungsform der vorliegenden Erfindung setzt man Katalysator in Form von Formkörpern ein, beispielsweise in Form von Tabletten oder Strängen.

Beispiele für besonders geeignete Dimensionen von Formkörpern sind Tabletten mit Abmessungen 6·3 mm, 3·3 mm, 2·2 mm, und Stränge mit einem Durchmesser im Bereich von 1,5 bis 3 mm.

Beispiele für Träger, die in partikulärer Form vorliegen, sind Pulver, die frei fließend oder suspendiert sein können.

Beispiele für Materialien, aus denen Träger, die in partikulärer Form vorliegen, sein können, sind Al₂O₃, SiO₂, Alumosilikate, Hydrotalcit, TiO₂, ZrO₂, Aktivkohle, insbesondere Al₂O₃, ZrO₂, und TiO₂.

Beispiele für Träger-freie katalytisch aktive Partikel (iii) sind Raney-Metalle, beispielsweise Raney-Kupfer, Raney-Nickel und Raney-Kobalt. Träger-freie katalytisch aktive Partikel können beispielsweise als Schwamm- oder Skelett-Katalysatoren vorliegen.

Katalysator kann neben Träger und Übergangsmetall ein oder mehrere Formmittel enthalten, beispielsweise Graphit oder Stearinsäure.

Beispiele für Übergangsmetalle, die als katalytisch aktive Spezies ("Haupt-Metall") in metallhaltigem Katalysator für das erfindungsgemäße Verfahren geeignet sind, sind Übergangsmetalle der Gruppen 4 bis 12 des Periodensystems, und zwar bevorzugt Übergangsmetalle der ersten Periode der Gruppen 4 bis 12 des Periodensystems, also von Ti bis Zn, sowie Übergangsmetalle der Gruppen 8 bis 11 sämtlicher Perioden des Periodensystems. Besonders bevorzugte Übergangsmetalle sind Co, Ni und Cu.

Übergangsmetall in Katalysator, der für das erfindungsgemäße Verfahren eingesetzt wird, kann dotiert sein, beispielsweise mit einem oder mehreren anderen Übergangsmetallen wie Zr oder Ti, oder mit Ca, mit Sn, mit Al oder mit Na. Unter Dotieren sollen hierbei Mengen an dotiertem Übergangsmetall bzw. Na, Al oder Ca verstanden werden, die man im Bereich von 0,1 bis 2 mol-% Übergangsmetall bzw. Na, Sn, Al oder Ca, bezogen auf Haupt-Metall, einbringt. Im Rahmen der vorliegenden Erfindung gelten jedoch aus der Gewinnung von Hauptmetall herrührende übliche Begleit-Spurenelemente als von einer Dotierung ausgenommen.

In einer Ausführungsform der vorliegenden Erfindung wählt man heterogene Katalysatoren aus Raney-Metallen und auf einem festen Träger aufgebrachtem Übergangsmetall. Bevorzugtes Übergangsmetall (Haupt-Metall) ist gewählt aus Ni, Cu und Co.

Zur Herstellung eines für das erfindungsgemäße Verfahren geeigneten Katalysators und zu seiner Lagerung setzt man Übergangsmetall in der Regel als Vorstufe, ein, nämlich als Verbindung, beispielsweise als Oxid, Hydroxid oder Oxidhydroxid, oder als Legierung, und aktiviert den Katalysator vor der Durchführung des erfindungsgemäßen Verfahrens oder *in situ*, vorzugsweise durch Reduktion oder durch Entfernung von mindestens einer Komponente der betreffenden Legierung. Vorzugsweise liegt Übergangsmetall in heterogenem Katalysator während der Durchführung des erfindungsgemäßen Verfahrens anteilig zumindest zeitweise in der Oxidationsstufe null vor.

In einer Ausführungsform der vorliegenden Erfindung wählt man Katalysatoren aus solchen Materialien in partikulärer Form, deren Masse - jeweils bestimmt vor der Aktivierung mit Wasserstoff - enthält:
insgesamt im Bereich von 15 bis 80 Gew.-% sauerstoffhaltige Verbindung(en) des Aluminiums, berechnet als Al₂O₃, bevorzugt 30 bis 70 Gew.-%, besonders bevorzugt 35 bis 65 Gew.-%, insgesamt im Bereich von 5 bis 35 Gew.-% sauerstoffhaltige Verbindung(en) des Nickels, berechnet als NiO, bevorzugt im Bereich von 10 bis 30 Gew.-%, besonders bevorzugt im Bereich von 12 bis 28 Gew.-%, ganz besonders bevorzugt 15 bis 25 Gew.-%,
insgesamt im Bereich von 5 bis 35 Gew.-% sauerstoffhaltige Verbindung(en) des Kobalts, berechnet als CoO, bevorzugt im Bereich von 10 bis 30 Gew.-%, besonders bevorzugt im Bereich von 12 bis 28 Gew.-%, ganz besonders bevorzugt 15 bis 25 Gew.-%,
insgesamt im Bereich von 1 bis 20 Gew.-% sauerstoffhaltige Verbindung(en) des Kupfers, berechnet als CuO, bevorzugt 2 bis 18 Gew.-%, besonders bevorzugt 5 bis 15 Gew.-%, insgesamt im Bereich von 0,2 bis 5 Gew.-% sauerstoffhaltige Verbindung(en) des Zinns, berechnet als SnO, bevorzugt im Bereich von 0,4 bis 4,0 Gew.-%, besonders bevorzugt im Bereich von 0,6 bis 3,0 Gew.-%, ganz besonders bevorzugt im Bereich von 0,7 bis 2,5 Gew.-%.

In einer Ausführungsform der vorliegenden Erfindung wählt man Katalysatoren aus solchen Materialien in partikulärer Form, deren Masse - jeweils bestimmt vor der Aktivierung mit Wasserstoff - enthält:
22 bis 45 Gew.-%, bevorzugt 25 bis 40 Gew.-%, sauerstoffhaltige Verbindungen des Zirkoniums, berechnet als ZrO₂,
1 bis 30 Gew.-%, bevorzugt 2 bis 25 Gew.-%, besonders bevorzugt 5 bis 15 Gew.-%, sauerstoffhaltige Verbindungen des Kupfers, berechnet als CuO,
5 bis 50 Gew.-%, bevorzugt 15 bis 45 Gew.-%, besonders bevorzugt 25 bis 40 Gew.-%, sauerstoffhaltige Verbindungen des Nickels, berechnet als NiO,
5 bis 50 Gew.-% sauerstoffhaltige Verbindungen des Kobalts, berechnet als CoO,
0 bis 10 Gew.-% sauerstoffhaltige Verbindungen des Aluminiums und/oder Mangans, wobei das Gewichtsverhältnis von Zirkonium, berechnet als ZrO₂, zu Aluminium und/oder Mangan, berechnet als Al₂O₃ und/oder MnO₂, bevorzugt mindestens 2,5 beträgt, ganz besonders bevorzugt 0 Gew.-% sauerstoffhaltige Verbindungen des Aluminiums und/oder Mangans,
0 bis 5 Gew.-%, bevorzugt 0 Gew.-% sauerstoffhaltige Verbindungen des Molybdäns, berechnet als MoO₃.

In einer Ausführungsform der vorliegenden Erfindung wählt man Katalysatoren aus solchen Materialien in partikulärer Form, deren Masse - jeweils bestimmt vor der Aktivierung mit Wasserstoff - enthält:
50 bis 95 Gew.-%, bevorzugt 55 bis 85 Gew.-%, besonders bevorzugt 60 bis 80 Gew.-% sauerstoffhaltige Verbindungen des Zirkoniums, berechnet als ZrO₂,
5 bis 50 Gew.-%, bevorzugt 15 bis 45 Gew.-%, besonders bevorzugt 20 bis 40 Gew.-%, sauerstoffhaltige Verbindungen des Nickels, berechnet als NiO.

In einer Ausführungsform der vorliegenden Erfindung beträgt das Molverhältnis von Nickel zu Kupfer größer 1, besonders bevorzugt größer 1,2, ganz besonders bevorzugt im Bereich von 1,8 bis 8,5.

Bevorzugt enthält die katalytisch aktive Masse von im erfindungsgemäßen Verfahren eingesetzten Katalysator kein Rhenium, kein Ruthenium, kein Eisen und/oder kein Zink, jeweils weder in metallischer (Oxidationsstufe = 0) noch in ionischer (Oxidationsstufe ≠ 0).

In einer Ausführungsform der vorliegenden Erfindung hat für erfindungsgemäßes Verfahren geeigneter Katalysator eine BET-Oberfläche im Bereich von 1 bis 1000 m²/g, bevorzugt von 10 bis 500 m²/g, gemessen durch N₂-Adsorption nach DIN 66131.

Zur Herstellung von bevorzugten im erfindungsgemäßen Verfahren verwendeten Katalysatoren der Variante (i) und (ii) sind verschiedene Verfahren möglich. Geeignete Katalysatoren der Variante (i) und (ii) sind beispielsweise durch Verkneten pulvriger Mischungen der Hydroxide, Carbonate, Oxide und/oder anderer Salze der Komponenten mit Wasser und nachfolgendes Extrudieren und Tempern (Wärmebehandlung) der so erhaltenen Masse erhältlich.

Bevorzugt werden zur Herstellung der im erfindungsgemäßen Verfahren eingesetzten Katalysatoren der Variante (i) und (ii) Fällungsmethoden angewandt. So können bevorzugte Katalysatoren beispielsweise durch eine gemeinsame Fällung von Nickel-, Kobalt-, Kupfer- und Zinn-Komponenten aus einer diese Elemente enthaltenden wässrigen Salzlösung mittels Basen in Gegenwart einer Aufschlämmung einer schwerlöslichen, sauerstoffhaltigen Aluminium-, Titan-, Silizium- und/oder Zirkoniumverbindung (Präzipitat) und anschließendes Waschen, Trocknen und Calcinieren des erhaltenen Präzipitats erhalten werden. Als schwerlösliche, sauerstoffhaltige Aluminium-, Titan-, Silizium- und/oder Zirkoniumlverbindungen können beispielsweise deren Oxide, Oxidhydrate, Phosphate, -borate und -silikate Verwendung finden. Aufschlämmungen der schwerlöslichen sauerstoffhaltigen Aluminium-, Titan-, Silizium- und/oder Zirkoniumverbindungen kann man durch Suspendieren feinkörniger Pulver derartiger Verbindungen in Wasser unter kräftigem Rühren herstellen. Bevorzugt stellt man Aufschlämmungen von schwerlöslichen sauerstoffhaltigen Aluminium-, Titan-, Silizium- bzw. Zirkoniumverbindungen durch Ausfällen der entsprechenden schwerlöslichen schwerlöslichen sauerstoffhaltigen Aluminium-, Titan-, Silizium- und Zirkoniumverbindungen aus wässrigen Lösungen von Aluminium-, Titan-, Silizium- bzw. Zirkoniumverbindungen mittels Base her.

Bevorzugt werden im erfindungsgemäßen Verfahren eingesetzte Katalysatoren der Variante (i) und (ii) über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Base - beispielsweise Natriumcarbonat, Natriumhydroxid, Kaliumcarbonat oder Kaliumhydroxid - versetzt, bis die Fällung vollständig ist. Es kann auch mit Alkalimetall-freien Basen wie Ammoniak, Ammoniumcarbonat, Ammoniumhydrogencarbonat, Ammoniumcarbamat, Ammoniumoxalat, Ammoniummalonat, Urotropin, Harnstoff, etc. gearbeitet werden. Die Art der verwendeten Salze ist im Allgemeinen nicht kritisch: Da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fällungsreaktionen erhaltenen Niederschläge sind im Allgemeinen chemisch uneinheitlich und bestehen u.a. aus Mischungen der Oxide, Oxidhydrate, Hydroxide, Carbonate und unlöslichen und basischen Salze der eingesetzten Metalle. Es kann sich für die Filtrierbarkeit der Niederschläge als günstig erweisen, wenn sie gealtert werden, d. h. wenn man sie noch einige Zeit nach der Fällung, gegebenenfalls in Wärme oder unter Durchleiten von Luft, sich selbst überlässt.

Die nach Fällungsverfahren erhaltenen Niederschläge kann man zu im erfindungsgemäßen Verfahren eingesetzten Katalysatoren der Variante (i) und (ii) nach an sich bekannten Methoden weiterverarbeiten. Zunächst wäscht man die Niederschläge. Über die Dauer des Waschens und über die Temperatur und Menge des Waschwassers kann der Gehalt an Alkalimetall, das durch die als Fällungsmittel eventuell verwendete (Mineral)base zugeführt wurde, beeinflusst werden. Im Allgemeinen wird durch Verlängerung des Waschens oder Erhöhung der Temperatur des Waschwassers der Gehalt an Alkalimetall abnehmen. Nach dem Waschen kann man die Niederschläge im Allgemeinen bei 80 bis 200 °C, vorzugsweise bei 100 bis 150 °C, trocknen und danach calcinieren. Das Calcinieren kann man bei Temperaturen zwischen 300 und 800 °C, vorzugsweise bei 400 bis 600 °C, insbesondere bei 420 bis 550 °C ausführen.

In einer anderen Ausführungsform kann man im erfindungsgemäßen Verfahren eingesetzten Katalysatoren der Variante (i) und (ii) durch Tränkung (Imprägnieren) von Aluminiumoxid (Al₂O₃), Titandioxid (TiO₂), Siliziumdioxid (SiO₂), Zirkoniumdioxid (ZrO₂), das beispielsweise in Form von Pulver oder Formkörpern, wie Strängen, Tabletten, Kugeln oder Ringen, vorliegt, oder von Mischungen von mindestens zwei der vorstehend genannten Oxide mit Übergangsmetallsalzösung herstellen.

Aluminiumoxid wird beispielsweise in der amorphen, gamma-, theta- und/oder delta- Form, als Aluminiumoxohydroxid (Böhmit), bevorzugt in der gamma-Form eingesetzt.

Zirkoniumdioxid kann man beispielsweise in amorpher, monokliner, tetragonaler oder kubischer Modifikation einsetzen, bevorzugt sind die monokline, die tetragonale und die kubische Modifikation. Besonders bevorzugt ist die monokline Modifikation.

Die Herstellung von Formkörpern kann nach an sich bekannten Methoden erfolgen.

In einer Ausführungsform der vorliegenden Erfindung setzt man 0,1 bis 120 Gew.-% Katalysator ein, bezogen auf α-Hydroxycarbonsäure.

In einer Ausführungsform des erfindungsgemäßen Verfahrens führt man es bei einer Temperatur im Bereich von 150 bis 280°C, bevorzugt 170 bis 250°C durch.

In einer Ausführungsform des erfindungsgemäßen Verfahrens führt man es bei einem Druck im Bereich von 10 bis 300 bar, bevorzugt 100 bis 250 bar, besonders bevorzugt von 150 bis 200 bar durch.

Das erfindungsgemäße Verfahren kann man absatzweise, kontinuierlich oder semikontinuierlich durchführen.

In einer Ausführungsform der vorliegenden Erfindung kann man die gesamte Reaktionsmischung oder bestimmte Komponenten der Reaktionsmischung im Kreis führen, beispielsweise Stickstoffhaltige Verbindung (c), insbesondere Ammoniak, oder Lösung von α-Hydroxycarbonsäure.

In einer Ausführungsform der vorliegenden Erfindung führt man das erfindungsgemäße Verfahren absatzweise bei im wesentlichen konstanter Temperatur durch, beispielsweise bei einer Temperatur, die während des erfindungsgemäßen Verfahrens um 10°C oder weniger schwankt, vorzugsweise um 5°C oder weniger.

In einer Ausführungsform der vorliegenden Erfindung führt man das erfindungsgemäße Verfahren über eine Dauer im Bereich von 1 Minute bis 48 Stunden durch. Wenn man das erfindungsgemäße Verfahren kontinuierlich durchzuführen wünscht, so soll unter der Dauer die mittlere Verweilzeit verstanden werden.

In einer Ausführungsform der vorliegenden Erfindung führt man das erfindungsgemäße Verfahren als Suspension von mindestens einem Katalysator nach Variante (i) oder (iii) durch, und zwar mit einer Reaktionsdauer im Bereich von 1 bis 48 Stunden, bevorzugt 2 bis 24 Stunden.

In einer anderen Ausführungsform der vorliegenden Erfindung führt man das erfindungsgemäße Verfahren mit einem Katalysator gemäß Variante (ii) durch, und zwar mit einer Reaktionsdauer im Bereich von 1 Minute bis 10 Stunden, bevorzugt 30 Minuten bis 5 Stunden.

Bei der Berechnung der Zeit sollen Zeiträume, die man zu Tätigkeiten wie Aufheizen, Abkühlen, Aufarbeitung der Reaktionsmischung, Isolierung von racemischer α-Aminosäure, Entspannen oder Aktivieren des Katalysators verwendet, nicht mit berücksichtigt werden.

In einer Ausführungsform der vorliegenden Erfindung kann man zur Durchführung des erfindungsgemäßen Verfahrens mischen, beispielsweise durch Rühren, Schütteln, Wälzen, Umpumpen, Pumpen durch statische Mischer oder pneumatisches Mischen.

Ohne dass einer bestimmten Theorie der Vorzug gegeben werden soll, so wird vermutet, dass im Verlaufe des erfindungsgemäßen Verfahrens α-Hydroxycarbonsäure zunächst zur α-Ketocarbonsäure oxidiert, also dehydriert, wird, die dann zur entsprechenden α-Iminocarbonsäure umgewandelt und dann zu racemischer α-Aminocarbonsäure reduziert wird.

Man erhält eine Reaktionsmischung, die Wasser und racemische α-Aminosäure bzw. Salz von racemischer α-Aminosäure enthält und weitere Bestandteile aufweisen kann, beispielsweise Katalysator(reste), Ausgangsmaterial wie α-Hydroxycarbonsäure oder Stickstoffverbindung (c), insbesondere Ammoniak, oder weiterhin Zersetzungsprodukte von α-Hydroxycarbonsäure, beispielsweise Propionsäure, Essigsäure oder Ameisensäure.

In einer Ausführungsform der vorliegenden Erfindung arbeitet man die entstehende Reaktionsmischung auf. In einer speziellen Ausführungsform der vorliegenden Erfindung isoliert man racemische α-Aminosäure bzw. ein Salz von racemischer α-Aminosäure.

Zur Aufarbeitung kann man beispielsweise eine oder mehrere folgenden Tätigkeiten ausführen:
(i) Katalysator deaktivieren,
(ii) Katalysator, der aktiv oder deaktiviert sein kann, abtrennen, beispielsweise durch Filtrieren, beispielsweise Kuchenfiltration oder Querstromfiltration, oder durch Sedimentation oder Zentrifugieren,
(iii) Wasser und Stickstoffverbindung (c), insbesondere Ammoniak, ganz oder teilweise entfernen, beispielsweise durch Verdampfen, Abdestillieren oder Sprühtrocknen,
(iv) Stickstoffverbindung (c) oder insbesondere Ammoniak mit Säure neutralisieren, insbesondere mit Brönsted-Säure, beispielsweise mit Schwefelsäure oder Salzsäure,
(v) Nebenprodukte abtrennen, die beispielsweise durch Reduktion von eingesetzter α-Hydroxycarbonsäure entstehen können,
(vi) pH-Wert einstellen, beispielsweise mit Brönsted-Säure oder Brönsted-Base,
(vii) α-Aminosäure von nicht umgesetzter α-Hydroxycarbonsäure mittels Ionenaustauscher abtrennen.

In einer Ausführungsform der vorliegenden Erfindung kristallisiert man anfallende racemische α-Aminosäure bzw. Salz von racemischer α-Aminosäure zur Aufreinigung um. Zum Umkristallisieren kann man verschiedene Lösungsmittel verwenden. Geeignet sind beispielsweise Wasser und wasserhaltige Gemische, beispielsweise Gemische von Wasser mit Ethanol. Bevorzugt kristallisiert man aus Wasser oder wässrigen Basen um, die einen pH-Wert im Bereich von 7,1 bis 14 aufweisen, bevorzugt 9 bis 12. Geeignete wässrige Basen sind verdünnte Kalilauge und insbesondere verdünnte Natronlauge.

Man kann ein- oder mehrmals umkristallisieren. Kristallisierte racemische α-Aminosäure bzw. kristallisiertes Salz von racemischer α-Aminosäure kann man von der Mutterlauge abtrennen, beispielsweise durch Dekantieren oder Filtrieren oder Kombination von Filtrieren und Dekantieren.

In einer Ausführungsform erhält man reine racemische α-Aminosäure bzw. reines Salz von racemischer α-Aminosäure, beispielsweise reines Natriumsalz oder reines Kaliumsalz, oder partiell neutralisierte reine racemische α-Aminosäure.

In einer anderen Ausführungsform der vorliegenden Erfindung erhält man Gemische, enthaltend
(a) im Bereich von 91 bis 99,9 Gew.-% einer racemischen α-Aminosäure, bevorzugt 95 bis 99,9 Gew.-%,
(b) im Bereich von 0,1 bis 9 Gew.-% der korrespondierenden α-Hydroxycarbonsäure, gewählt aus Hydroxyessigsäure, Milchsäure, Äpfelsäure, 2-Hydroxyglutarsäure, Isocitronensäure, Tartronsäure und Weinsäure, bevorzugt 0,5 bis 5 Gew.-%,
in jeweils reiner Form oder partiell oder vollständig neutralisiert, vorzugsweise mit Kalium oder Ammonium oder insbesondere mit Natrium,
wobei Angaben in Gew.-% jeweils auf das gesamte Gemisch bezogen sind,
und die ebenfalls Gegenstand der vorliegenden Erfindung sind.

Racemische α-Aminosäure wird gewählt aus Glycin und jeweils dem Racemat von Alanin, Asparaginsäure, Glutaminsäure, -Aminopropan-1,2,3-tricarbonsäure, 2-Aminomalonsäure, 2-Amino-3-hydroxybersteinsäure und 2,3-Diaminobernsteinsäure.

Korrespondierende α-Hydroxycarbonsäure (b) kann enatiomerenangereichert sein oder vorzugsweise racemisch.

Der Nachweis, dass es sich bei racemischer α-Aminosäure (a) um das Racemat handelt, gelingt beispielsweise durch Polarimetrie.

In einer bevorzugten Ausführungsform der vorliegenden Erfindung wird in erfindungsgemäßen Gemischen
racemische α-Aminosäure (a) gewählt aus racemischem α-Alanin und α-Hydroxycarbonsäure (b) gewählt aus vorzugsweise racemischer Milchsäure in jeweils reiner Form oder partiell oder vollständig neutralisiert, vorzugsweise mit Kalium oder Ammonium oder insbesondere Natrium.

Erfindungsgemäße Gemische kann man beispielsweise nach dem erfindungsgemäßen Verfahren herstellen.

Ein weiterer Gegenstand der vorliegenden Erfindung ist die Verwendung von erfindungsgemäßen Gemischen zur Herstellung von Komplexierungsmitteln. Ein weiterer Gegenstand der vorliegenden Erfindung ist ein Verfahren zur Herstellung von Komplexierungsmitteln unter Verwendung von mindestens einem erfindungsgemäßen Gemisch.

Erfindungsgemäße Gemische kann man beispielsweise einsetzen, um Komplexierungsmittel herzustellen. Beispielsweise kann man Gemische von racemischem α-Alanin und racemischer Milchsäure einsetzen, um racemische Methylglycindiessigsäure (MGDA) herzustellen, beispielsweise durch Ethoxylierung und anschließende Oxidation der alkoholischen CH₂-OH-Gruppen, oder durch Strecker-Synthese mit HCN/HCHO. Der Anteil von Milchsäure, insbesondere an racemischer Milchsäure, stört in einem solchen Fall bei der Herstellung von MGDA nicht. Man erhält eine Mischung von Milchsäure und racemischem MGDA, als reine Säure oder vollständig oder partiell neutralisiert, beispielsweise mit Natrium oder Kalium.

Ein weiterer Gegenstand der vorliegenden Erfindung sind Formulierungen, enthaltend mindestens ein erfindungsgemäßes Gemisch.

Ein spezieller Gegenstand der vorliegenden Erfindung ist eine Mischung, enthaltend
(a) im Bereich von 0,1 bis 5 Gew.-% Milchsäure, vorzugsweise als Racemat,
(b) im Bereich von racemische 95 bis 99,9 Gew.-% N,N-Methylglycindiessigsäure,
in jeweils reiner Form oder partiell oder vollständig neutralisiert, vorzugsweise mit Kalium oder Ammonium oder insbesondere Natrium.

Die Erfindung wird durch Arbeitsbeispiele verdeutlicht.

Angaben in % sind Gew.-%, wenn nicht ausdrücklich anderes angegeben ist.
Auch die nicht-aktivierten Katalysatoren werden im Rahmen der Beschreibung als "Katalysator" bezeichnet.

### I. Herstellung von Katalysatoren

### I.1 Herstellung von Katalysator I.1

In ein auf 65°C geheiztes Rührgefäß wurden unter Rühren gleichzeitig eingeleitet:
- eine wässrige Lösung aus Nickelnitrat, Kobaltnitrat, Kupfernitrat, Aluminiumnitrat und Zinn(II)chlorid, die umgerechnet 3,9 % Ni, 3,9 % Co, 1,9 % Cu, 5,5 % Al₂O₃ und 0,5 % Sn enthielt ("Übergangsmetallsalzlösung"), und
- eine 20 Gew.-% wässrige Natriumcarbonatlösung.

Dazu wurde die Übergangsmetallsalzlösung in einem konstanten Strom eingeleitet. Die Dosierung der 20 Gew.-% wässrigen Natriumcarbonatlösung wurde so geregelt, dass der pH-Wert von 5,7, gemessen mit einer Glaselektrode, konstant gehalten wurde. Durch Ausfällung von Nickel-, Kobalt-, Aluminium-, Kupfer- und Zinnverbindungen entstand eine Suspension. Die Temperatur der Suspension war 65°C. Nach beendeter Fällung wurde eine Stunde lang Luft eingeblasen, danach wurde der pH-Wert der Suspension mit Natriumcarbonatlösung auf den Wert 7,4 eingestellt. Die so erhaltene Suspension wurde filtriert und der Filterkuchen mit vollentsalztem Wasser gewaschen, bis die elektrische Leitfähigkeit des Filtrats ca. 20 mS betrug. Danach wurde der Filterkuchen bei einer Temperatur von 150 °C in einem Trockenschrank getrocknet. Das auf diese Weise erhaltene Hydroxidcarbonatgemisch wurde nun bei einer Temperatur von 500 °C über 4 Stunden kalziniert. Die Katalysatormasse wurde anschließend mit 3 Gew.-% Graphit vermischt und zu Tabletten 3·3 mm verformt. Die auf dieser Weise erhaltenen Tabletten wurden bei einer Temperatur von 280 bis 300 °C über mindestens 12 Stunden in Wasserstoff reduziert. Die Passivierung des reduzierten Katalysators wurde bei Raumtemperatur in "verdünnter" Luft (O₂-Gehalt von maximal 5 Vol.-%, Luft in N₂) durchgeführt. Die Zusammensetzung des so erhaltenen Katalysators I.1 ist in Tabelle I dargestellt.

### I.2 Herstellung des Katalysators I.2

In ein auf 65°C geheiztes Rührgefäß wurden unter Rühren gleichzeitig eingeleitet:
- eine wässrige Lösung aus Nickelnitrat, Kobaltnitrat, Kupfernitrat, Zirkonacetat, die umgerechnet 7 Gew.-% NiO, 7 Gew.-% CoO, 3,25 Gew.-%CuO und 7,75 Gew.-% ZrO2 enthielt ("Übergangsmetallsalzlösung") und
- eine 20 Gew.-% wässrige Natriumcarbonatlösung.

Dazu wurde die Übergangsmetallsalzlösung in einem konstanten Strom eingeleitet. Die Dosierung der 20 Gew.-% wässrigen Natriumcarbonatlösung wurde so geregelt, dass der pH-Wert von 7,5, gemessen mit einer Glaselektrode, konstant gehalten wurde. Durch Ausfällung von Nickel-, Cobalt-, Kupfer- und Zirkonverbindungen entstand eine Suspension. Die Temperatur der Suspension war 65°C. Nach beendeter Fällung wurde die so erhaltene Suspension filtriert und der Filterkuchen mit vollentsalztem Wasser gewaschen, bis die elektrische Leitfähigkeit des Filtrats ca. 20 mS betrug. Danach wurde der Filterkuchen bei einer Temperatur von 120 °C in einem Trockenschrank getrocknet. Das auf diese Weise erhaltene Hydroxidcarbonatgemisch wurde nun bei einer Temperatur von 400 °C über 2 Stunden kalziniert. Die Katalysatormasse wurde anschließend zu Tabletten 3·3 mm verformt. Die auf diese Weise erhaltenen Tabletten wurden bei einer Temperatur von 280 bis 300 °C über mindestens 12 Stunden in Wasserstoff reduziert. Die Passivierung des reduzierten Katalysators wurde bei Raumtemperatur in "verdünnter" Luft (O₂-Gehalt von maximal 5 Vol.-%, Luft in N₂) durchgeführt. Die Zusammensetzung des so erhaltenen Katalysators I.1 ist in Tabelle I dargestellt.

**Tabelle I: Zusammensetzung von Katalysatoren I.1 und I.2**

| Katalysator *) | Ni | Co | Cu | Sn | BET | Träger |
|---|---|---|---|---|---|---|
| | % | % | % | % | m²/g | |
| I.1 | 18,6 | 17,3 | 10,6 | 1,1 | 187 | Al₂O₃ |
| I.2 | 28 | 28 | 13 | -- | 75 | ZrO₂ |

| | | | | | | |
|---|---|---|---|---|---|---|
| *): Katalysatorzusammensetzung in Gew.-%; Rest bis zu 100 Gew.-% ist der Träger. | | | | | | |

### II. Herstellung von α-Alanin (racemisch)

### II.1 Herstellung von α-Alanin mit einem Raney-Nickel-Katalysator

In einem 300-ml-Autoklaven wurden 10 g Raney-Nickel als Katalysator, 74 g einer 36 Gew.-% wässrigen Lösung von Natriumsalz von L-Milchsäure und 45 g flüssiges Ammoniak vorgelegt. Man presste 20 bar Wasserstoff auf und erhitzte auf 200°C. Anschließend erhöhte man durch Aufpressen von weiterem Wasserstoff den Druck auf 200 bar. Danach rührte man 24 Stunden bei 200 bar Wasserstoff und 200°C. Nach 16 Stunden entnahm man ein Aliquot und bestimmte den Umsatz zu 64%, bezogen auf Milchsäure, und bestimmt durch ¹H NMR-Spektroskopie. Nach insgesamt 24 Stunden kühlte man auf Zimmertemperatur ab, entspannte, filtrierte den Katalysator ab und destillierte 45 g des Wassers sowie nicht umgesetzten Ammoniak ab.

Man erhielt ein Gemisch, enthaltend 79 mol-% racemisches α-Alanin und 21 mol-% racemische Milchsäure, jeweils als Natriumsalz. Die Restfeuchte betrug 20 Gew.-%, bezogen auf Summe an racemischem α-Alanin und racemischer Milchsäure, jeweils als Natriumsalz.

### II.2 Herstellung von α-Alanin mit einem Raney-Nickel-Katalysator

In einem 300-ml-Autoklaven wurden 10 g Raney-Nickel als Katalysator, 74 g einer 36 Gew.-% wässrigen Lösung von Natriumsalz von L-Milchsäure und 45 g flüssiges Ammoniak vorgelegt. Man presste 20 bar Wasserstoff auf und erhitzte auf 210°C. Anschließend erhöhte man durch Aufpressen von weiterem Wasserstoff den Druck auf 200 bar. Danach rührte man 24 Stunden bei 200 bar Wasserstoff und 210°C. Nach 16 Stunden entnahm man ein Aliquot und bestimmte den Umsatz zu 89%, bezogen auf Milchsäure, und bestimmt durch ¹H NMR-Spektroskopie. Nach insgesamt 24 Stunden kühlte man auf Zimmertemperatur ab, entspannte, filtrierte den Katalysator ab und destillierte 45 g des Wassers sowie nicht umgesetzten Ammoniak ab.

Man erhielt ein erfindungsgemäßes Gemisch GM-AM.1, enthaltend 92 mol-% racemisches α-Alanin und 8 mol-% racemische Milchsäure, jeweils als Natriumsalz. Das entspricht einem Gewichtsverhältnis von 91,9 Gew.-% racemischem α-Alanin zu 8,1 Gew.-% racemischer Milchsäure, bezogen jeweils auf die freie Säure.

GM-AM.1 ließ sich sehr gut zur Herstellung von Mischungen aus (±)-MGDA und racemischer Milchsäure verarbeiten.

### II.3 Herstellung von α-Alanin mit Hilfe eines Ni-Co-Cu-Sn-Katalysators

Man füllte 10 g Katalysator I.1 in einen Katalysatorkorb aus Edelstahl. Man setzte den so gefüllten Katalysatorkorb in einen 300-ml-Autoklaven und behandelte mit Wasserstoff bei 250°C über einen Zeitraum von 24 Stunden. Dadurch wurde der Katalysator aktiviert. Man entspannte, kühlte auf Zimmertemperatur ab und gab 74 g einer 36 Gew.-% wässrigen Lösung von Natriumsalz von L-Milchsäure und 45 g flüssigen Ammoniak zu. Man presste 20 bar Wasserstoff auf und erhitzte auf 200°C Anschließend erhöhte man durch Aufpressen von weiterem Wasserstoff den Druck auf 200 bar. Danach rührte man 24 Stunden bei 200 bar Wasserstoff und 200°C. Nach 16 Stunden entnahm man ein Aliquot und bestimmte den Umsatz zu 64%, bezogen auf Milchsäure, und bestimmt durch ¹H NMR-Spektroskopie. Nach insgesamt 24 Stunden kühlte man auf Zimmertemperatur ab, entspannte, entfernte den Katalysatorkorb mitsamt Katalysator und destillierte 45 g Wasser sowie nicht umgesetzten Ammoniak ab.

Man erhielt ein Gemisch, enthaltend 79 mol-% racemisches α-Alanin und 21 mol-% racemische Milchsäure, jeweils als Natriumsalz. Die Restfeuchte betrug 15 Gew.-%, bezogen auf Summe an racemischem α-Alanin und racemischer Milchsäure, jeweils als Natriumsalz.

Eine Analyse des Gasraums oberhalb der Reaktionsmischung ergab einen Anteil von 0,15 Vol.-% Methan.

### II.4 Herstellung von α-Alanin mit Hilfe eines Ni-Co-Cu-Sn-Katalysators

Man füllte 10 g Katalysator I.1 in einen Katalysatorkorb aus Edelstahl. Man setzte den so gefüllten Katalysatorkorb in einen 300-ml-Autoklaven und behandelte mit Wasserstoff bei 250°C über einen Zeitraum von 24 Stunden. Dadurch wurde der Katalysator aktiviert. Man entspannte, kühlte auf Zimmertemperatur ab und gab 74 g einer 36 Gew.-% wässrigen Lösung von Natriumsalz von L-Milchsäure und 45 g flüssigen Ammoniak zu. Man presste 20 bar Wasserstoff auf und erhitzte auf 210°C Anschließend erhöhte man durch Aufpressen von weiterem Wasserstoff den Druck auf 200 bar. Danach rührte man 24 Stunden bei 200 bar Wasserstoff und 210°C. Nach 16 Stunden entnahm man ein Aliquot und bestimmte den Umsatz zu 85%, bezogen auf Milchsäure, und bestimmt durch ¹H NMR-Spektroskopie. Nach insgesamt 24 Stunden kühlte man auf Zimmertemperatur ab, entspannte, entfernte den Katalysatorkorb mitsamt Katalysator und destillierte 45 g Wasser sowie nicht umgesetzten Ammoniak ab.

Man erhielt ein erfindungsgemäßes Gemisch, enthaltend 92 mol-% racemisches α-Alanin und 8 mol-% racemische Milchsäure, jeweils als Natriumsalz. Es entsprach in seiner Zusammensetzung erfindungsgemäßem Gemisch GM-AM.1 aus Beispiel 2.

### II.5 Kontinuierliche Herstellung von α-Alanin mit Hilfe eines Ni-Co-Cu-Katalysators

Man füllte 500 ml des Katalysators I.2 in einen Festbettreaktor, Abmessungen: Länge 2 m, kreisförmiger Durchmesser: 3 cm, mit Rückführpumpe. Man behandelte den Katalysator mit Wasserstoff bei 280°C drucklos über einen Zeitraum von 24 Stunden. Dadurch wurde der Katalysator aktiviert. Anschließend betrieb man den Festbettreaktor so, dass man das Festbett kontinuierlich von unten nach oben mit 91 g/h einer 55 Gew.-% wässrigen Lösung von Natriumsalz von L-Milchsäure, 227 g/h gasförmigem Ammoniak und 100 Nl/h Wasserstoff betrieben. Die Rückführpumpe förderte 502 g/h des Reaktionsgemisches. Man stellte eine Temperatur von 210°C und einen Druck von 200 bar ein. Nach dem Entspannen erhielt man eine wässrige Lösung, enthaltend die Natriumsalze von racemischem α-Alanin und racemischer Milchsäure im Molverhältnis 83:17.

### II.6 Kontinuierliche Herstellung von α-Alanin mit Hilfe eines Ni-Co-Cu-Katalysators

Man füllte 500 ml des Katalysators I.2 in einen Festbettreaktor, Abmessungen: Länge 2 m, kreisförmiger Durchmesser: 3 cm. Man behandelte den Katalysator mit Wasserstoff bei 280°C drucklos über einen Zeitraum von 24 Stunden. Dadurch wurde der Katalysator aktiviert. Anschließend betrieb man den Festbettreaktor so, dass man das Festbett kontinuierlich von unten nach oben mit 69 g/h einer 60 Gew.-% wässrigen Lösung von Natriumsalz von L-Milchsäure, 77 g/h gasförmigem Ammoniak und 50 Nl/h Wasserstoff betrieben. Man stellte eine Temperatur von 200°C und einen Druck von 50 bar ein. Man entspannte über ein Regelventil und erhielt ein erfindungsgemäßes Gemisch GM-AM.6, enthaltend racemisches α-Alanin und racemische Milchsäure, jeweils als Natriumsalz, im Molverhältnis 92:8.

GM-AM.6 ließ sich sehr gut zur Herstellung von Mischungen aus (±)-MGDA und racemischer Milchsäure verarbeiten.

## Patentansprüche

1. Verfahren zur Herstellung von racemischen α-Aminosäuren oder von Glycin, **dadurch gekennzeichnet, dass** man die korrespondierende α-Hydroxycarbonsäure, gewählt aus Hydroxyessigsäure, Milchsäure, Äpfelsäure, α-Hydroxyglutarsäure, Isocitronensäure, Tartronsäure und Weinsäure, oder mindestens ein Salz der korrespondierenden α-Hydroxycarbonsäure in Gegenwart von mindestens einem heterogenen Katalysator, der mindestens ein Übergangsmetall enthält, in Gegenwart von Wasserstoff mit mindestens einer Stickstoffverbindung (c) umsetzt, wobei man Stickstoffverbindung (c) wählt aus primären und sekundären Aminen und Ammoniak.

2. Verfahren nach Anspruch 1, **dadurch gekennzeichnet, dass** man heterogene Katalysatoren wählt aus Raney-Metallen und auf einem festen Träger aufgebrachtem Übergangsmetall.

3. Verfahren nach Anspruch 1 oder 2, **dadurch gekennzeichnet, dass** man Übergangsmetall wählt aus Ni, Cu und Co.

4. Verfahren nach einem der Ansprüche 1 bis 3, **dadurch gekennzeichnet, dass** Übergangsmetall in heterogenem Katalysator während des Verfahrens zumindest zeitweise in der Oxidationsstufe null vorliegt.

5. Verfahren nach einem der Ansprüche 1 bis 4, **dadurch gekennzeichnet, dass** man Stickstoffverbindung (c) wählt aus Ammoniak.

6. Verfahren nach einem der Ansprüche 1 bis 5, **dadurch gekennzeichnet, dass** man es bei einer Temperatur im Bereich von 150 bis 280°C durchführt.

7. Verfahren nach einem der Ansprüche 1 bis 6, **dadurch gekennzeichnet, dass** man α-Hydroxycarbonsäure wählt aus racemischer Milchsäure, (-)-Milchsäure und (+)-Milchsäure.

8. Verfahren nach einem der Ansprüche 1 bis 7, **dadurch gekennzeichnet, dass** man Salze von α-Hydroxycarbonsäuren wählt aus Alkalimetallsalzen und Ammoniumsalzen von α-Hydroxycarbonsäure.

9. Verfahren nach einem der Ansprüche 1 bis 8, **dadurch gekennzeichnet, dass** man es bei einem Druck im Bereich von 10 bis 300 bar durchführt.

10. Verfahren nach einem der Ansprüche 1 bis 9, **dadurch gekennzeichnet, dass** man es in wässrigem Medium durchführt.

11. Verfahren nach einem der Ansprüche 1 bis 10, **dadurch gekennzeichnet, dass** man zur Aufreinigung der anfallenden racemischen α-Aminosäure oder von Glycin umkristallisiert.

12. Gemisch, enthaltend
(a) im Bereich von 91 bis 99,9 Gew.-% einer racemischen α-Aminosäure, gewählt aus Glycin und jeweils dem Racemat von Alanin, Asparaginsäure, Glutaminsäure, 1-Aminopropan-1,2,3-tricarbonsäure, 2-Aminomalonsäure, 2-Amino-3-hydroxybernsteinsäure und 2,3-Diaminobernsteinsäure,
(b) im Bereich von 0,1 bis 9 Gew.-% der korrespondierenden α-Hydroxycarbonsäure,
in jeweils reiner Form oder partiell oder vollständig neutralisiert,
wobei Angaben in Gew.-% jeweils auf das gesamte Gemisch bezogen sind.

13. Gemisch nach Anspruch 12, in denen racemische α-Aminosäure (a) gewählt wird aus racemischem α-Alanin und racemische α-Hydroxycarbonsäure (b) gewählt wird aus racemischer Milchsäure.

14. Formulierungen, enthaltend mindestens ein Gemisch nach Anspruch 12 oder 13.

15. Verwendung von Gemischen nach Anspruch 12 oder 13 zur Herstellung von Komplexierungsmitteln.

## Claims

1. A process for the preparation of racemic α-amino acids or of glycine, wherein the corresponding α-hydroxycarboxylic acid, selected from hydroxyacetic acid, lactic acid, malic acid, α-hydroxyglutaric acid, isocitric acid, tartronic acid and tartaric acid, or at least one salt of the corresponding α-hydroxycarboxylic acid is reacted in the presence of at least one heterogeneous catalyst which comprises at least one transition metal, in the presence of hydrogen with at least one nitrogen compound (c), where nitrogen compound (c) is selected from primary and secondary amines and ammonia.

2. The process according to claim 1, wherein heterogeneous catalysts are selected from Raney metals and transition metal applied to a solid support.

3. The process according to claim 1 or 2, wherein the transition metal is selected from Ni, Cu and Co.

4. The process according to any one of claims 1 to 3, wherein the transition metal in the heterogeneous catalyst is present during the process at least for part of the time in oxidation state zero.

5. The process according to any one of claims 1 to 4, wherein the nitrogen compound (c) is selected from ammonia.

6. The process according to any one of claims 1 to 5, which is carried out at a temperature in the range from 150 to 280°C.

7. The process according to any one of claims 1 to 6, wherein the α-hydroxycarboxylic acid is selected from racemic lactic acid, (-)-lactic acid and (+)-lactic acid.

8. The process according to any one of claims 1 to 7, wherein salts of α-hydroxycarboxylic acids are selected from alkali metal salts and ammonium salts of α-hydroxycarboxylic acid.

9. The process according to any one of claims 1 to 8, which is carried out at a pressure in the range from 10 to 300 bar.

10. The process according to any one of claims 1 to 9, which is carried out in an aqueous medium.

11. The process according to any one of claims 1 to 10, which comprises recrystallization for the purposes of purifying the racemic α-amino acid produced or glycine.

12. A mixture comprising
(a) in the range from 91 to 99.9% by weight of a racemic α-amino acid, selected from glycine and in each case the racemate of alanine, aspartic acid, glutamic acid, aminopropane - 1,2,3-tricarboxylic acid, 2-aminomalonic acid, 2-amino-3-hydroxysuccinic acid and 2,3-diaminosuccinic acid,
(b) in the range from 0.1 to 9% by weight of the corresponding α-hydroxycarboxylic acid,
in in each case pure form or partially or completely neutralized,
where data in % by weight are based in each case on the total mixture

13. The mixture according to claim 12, in which racemic α-amino acid (a) is selected from racemic α-alanine and racemic α-hydroxycarboxylic acid (b) is selected from racemic lactic acid.

14. A formulation comprising at least one mixture according to claim 12 or 13.

15. The use of mixtures according to claim 12 or 13 for producing complexing agents.

## Revendications

1. Procédé de fabrication d'acides α-aminés racémiques ou de glycine, **caractérisé en ce que** l'acide α-hydroxycarboxylique correspondant, choisi parmi l'acide hydroxyacétique, l'acide lactique, l'acide maltique, l'acide α-hydroxyglutarique, l'acide isocitrique, l'acide tartronique et l'acide tartrique, ou au moins un sel de l'acide α-hydroxycarboxylique correspondant, est mis en réaction en présence d'au moins un catalyseur hétérogène, qui contient au moins un métal de transition, en présence d'hydrogène avec au moins un composé d'azote (c), le composé d'azote (c) étant choisi parmi les amines primaires et secondaires et l'ammoniac.

2. Procédé selon la revendication 1, **caractérisé en ce que** les catalyseurs hétérogènes sont choisis parmi les métaux de Raney et un métal de transition appliqué sur un support solide.

3. Procédé selon la revendication 1 ou 2, **caractérisé en ce que** le métal de transition est choisi parmi Ni, Cu et Co.

4. Procédé selon l'une quelconque des revendications 1 à 3, **caractérisé en ce que** le métal de transition dans le catalyseur hétérogène se présente au moins périodiquement à l'état d'oxydation zéro pendant le procédé.

5. Procédé selon l'une quelconque des revendications 1 à 4, **caractérisé en ce que** le composé d'azote (c) est choisi parmi l'ammoniac.

6. Procédé selon l'une quelconque des revendications 1 à 5, **caractérisé en ce qu'**il est réalisé à une température dans la plage allant de 150 à 280 °C.

7. Procédé selon l'une quelconque des revendications 1 à 6, **caractérisé en ce que** l'acide α-hydroxycarboxylique est choisi parmi l'acide lactique racémique, l'acide (-)-lactique et l'acide (+)-lactique.

8. Procédé selon l'une quelconque des revendications 1 à 7, **caractérisé en ce que** les sels d'acides α-hydroxycarboxyliques sont choisis parmi les sels de métaux alcalins et les sels d'ammonium d'acides α-hydroxycarboxyliques.

9. Procédé selon l'une quelconque des revendications 1 à 8, **caractérisé en ce qu'**il est réalisé à une pression dans la plage allant de 10 à 300 bar.

10. Procédé selon l'une quelconque des revendications 1 à 9, **caractérisé en ce qu'**il est réalisé en milieu aqueux.

11. Procédé selon l'une quelconque des revendications 1 à 10, **caractérisé en ce qu'**une recristallisation est effectuée pour la purification de l'acide α-aminé racémique ou de la glycine formé.

12. Mélange contenant :
(a) dans la plage allant de 91 à 99,9 % en poids d'un acide α-aminé racémique, choisi parmi la glycine et à chaque fois le racémat d'alanine, d'acide asparaginique, d'acide glutamique, d'acide aminopropane-1,2,3-tricarboxylique, d'acide 2-aminomalonique, d'acide 2-amino-3-hydroxysuccinique et d'acide 2,3-diaminosuccinique,
(b) dans la plage allant de 0, 1 à 9 % en poids de l'acide α-hydroxycarboxylique correspondant,
à chaque fois sous forme pure ou neutralisée en partie ou en totalité,
les données en % en poids se rapportant à chaque fois au mélange total.

13. Mélange selon la revendication 12, dans lequel l'acide α-aminé racémique (a) est choisi parmi l'α-alanine racémique et l'acide α-hydroxycarboxylique racémique (b) est choisi parmi l'acide lactique racémique.

14. Formulations, contenant au moins un mélange selon la revendication 12 ou 13.

15. Utilisation de mélanges selon la revendication 12 ou 13 pour la fabrication de complexants.
